# EUROPÄISCHE PATENTSCHRIFT

(11) **EP 2 403 519 B1**
(45) Veröffentlichungstag und Bekanntmachung des Hinweises auf die Patenterteilung: **15.11.2017**
(21) Anmeldenummer: 10709949.1
(22) Anmeldetag: 05.03.2010
(51) Int. Cl.: C07K 7/64, A61K 38/12, A61K 38/19

(54) **VERFAHREN ZUR VERMEIDUNG UND ZUR BEHANDLUNG EINER HYPERPERMEABILITÄT**
METHOD FOR PREVENTING AND TREATING HYPERPERMEABILITY
PROCÉDÉ POUR EMPÊCHER ET TRAITER UNE HYPERPERMÉABILITÉ

(30) Priorität: 05.03.2009 AT 3592009
(43) Veröffentlichungstag der Anmeldung: 11.01.2012
(73) Patentinhaber: APEPTICO Forschung und Entwicklung GmbH, 1150 Wien (AT)
(72) Erfinder: FISCHER, Bernhard, A-1160 Wien (AT); LUCAS, Rudolf, B-2630 Aartselaar (BE)
(74) Vertreter: Sonn & Partner Patentanwälte
(86) Internationale Anmeldenummer: PCT/AT2010/000056
(87) Internationale Veröffentlichungsnummer: WO 2010/099556

(56) Entgegenhaltungen:
- EP-A1- 1 264 599
- EP-A1- 2 009 023
- WO-A1-2006/013183
- WO-A1-2009/073909
- DE-T2- 60 004 340
- US-A1- 2007 154 482

## Beschreibung

Die vorliegende Erfindung betrifft Verfahren zur Vermeidung und zur Behandlung einer Hyperpermeabilität in Endothelzellen und Epithelzellen.

Endothelzellen und Epithelzellen übernehmen entscheidende Funktionen in allen Geweben und Organen des menschlichen und tierischen Körpers.

Das Endothelium besteht aus einer dünnen Schicht von Endothelzellen. Die Schicht aus Endothelzellen bildet u.a. die innere Oberfläche der Blutgefäße, wie Adern und Kapillaren, und die Barriere zwischen dem Blut und der äußeren Wand der Blutgefäße. Endothelzellen kleiden das gesamte Blutsystem aus, von den großen Blutgefäßen bis hin zu den kleinsten Kapillaren. Epithelzellen bilden ein- oder mehrlagige Zellschichten, die alle inneren und äußeren Körperoberflächen der menschlichen und tierischen Organe bedecken. Epithelzellen liegen dicht beieinander und sind reich an Zellkontakten. Bei Epithelzellen kann eine äußere, apikale Seite, die dem Äußeren oder dem Lumen zugewandt ist, und eine basale Seite unterschieden werden. Des Weiteren besitzen Epithelzellen einen Haftkomplex (Schlussleistenkomplex) bestehend aus Zonula occludens (Tight junction), Zonula adhaerens (Adhaerens junction) und Desmosom (Macula adhaerens), der zum einen eine physikochemische Barriere darstellt und zum anderen angrenzende Epithelzellen miteinander verbindet.

Für die physiologische Funktion aller tierischen und menschlichen Organe und Organellen ist die Unversehrtheit, insbesondere der begrenzenden Zellen und Zellschichten, äußerst wichtig. Kommt es zum Beispiel zur Verletzung der Endothelzellen, bzw. zur Verletzung des Endothelium der Blutgefäße, so kann Flüssigkeit aus den Blutgefäßen ausdringen und zu massiven Störungen in der Vitalität des gesamten Organismus führen.

Kommt es zum Beispiel zur Verletzung der Epithelzellen, bzw. zur Verletzung des Epitheliums von Organen, so kann sowohl aus den Organen Flüssigkeit austreten, oder auch Flüssigkeit eindringen, und somit die Funktionalität der Organe ernsthaft schädigen.

Durch eine Verletzung des Endothels und des Epithels kann es zu einer sogenannten Hyperpermeabilität kommen, d.h. zu einem unkontrollierten Durchtritt von Flüssigkeit aus Blutgefäßen und in lebenswichtigen Organen und Gewebe.

Neben mechanischen Ursachen kann eine Infektion oder die Einwirkung von Toxinen zu einer Hyperpermeabilität führen. Mikrobielle Toxine sind an Cholesterol bindende, Poren-formende Moleküle, welche von Gram-positiven Bakterien freigesetzt werden. Durch die Wirkung von Toxinen entstehen in Zellmembranen zuerst Poren und dann Makroporen. Dadurch werden Zellschichten durchlässig für Flüssigkeit und darin enthaltende Stoffe.

Bekannte Toxine sind u.a. das Listeriolysin aus Listeria monocytogenes, oder auch Pneumolysin aus Streptococcus pneumoniae. Diese Toxine können zur Bildung von Reaktiven Sauerstoffmolekülen in den Zellen führen. Die durch Toxine bedingten Reaktiven Sauerstoffmoleküle führen dann zu einer Schädigung des Endothels und des Epithels u.a. dadurch, dass die Barrierefunktion der Zellen geschädigt wird.

Zur Aufrechterhaltung der Barrierefunktion von Endothelzellschichten und Epithelzellschichten, sind die Zellen miteinander über Proteinfasern verbunden. Bestandteile solcher Proteinfasern ist z.B. die Myosin Light Chain. Durch eine Phosphorylierung der Myosin Light Chain kommt es jedoch zu Spannungen in den Zellen und den Zell-Zell Verbindungen, sowie zur Bildung von interzellulären Spalten, wodurch Flüssigkeit unkontrolliert ein- und auch ausfließen kann.

Eine weitere Komponente in der Regulation der Barriere-Funktion der Epithelzellen und Endothelzellen ist die Protein Kinase C. Für Protein Kinasen C sind mehrere Isoenzyme bekannt, z.B. Protein Kinase alpha und zeta. Diese Protein Kinase C I-soenzyme werden durch Reaktive Sauerstoffmoleküle, Wasserstoffperoxid, mikrobielle Toxine, wie Pneumolysin und Listeriolysin, und hydrophile Coronavirusproteine aktiviert. Aktivierte Protein Kinase C führt außerdem zu einer Verringerung der Expression des epithelialen Natriumkanals (ENaC), welcher für den Natrium- und Flüssigkeitstransport in Epithelzellen verantwortlich ist, und somit trägt aktivierte Protein Kinase C zur Herausbildung einer Hyperpermeabilität wesentlich bei.

Andere Ursachen zur Herausbildung einer Hyperpermeabilität in der Lunge sind z.B. Viren, wie Influenza-Viren, der "Severe Acute Respiratory Syndrome-associated Coronavirus (SARS-CoV)" oder der "Respiratory Syncytial Virus", welche zu einer Hyperpermeabilität des Endothel und Epithel, sowie zu einer atypischen Lungenentzündung führen können. Es ist bekannt, dass SARS-CoV Proteine durch die Aktivierung der Protein Kinase C Isoform zu einer Verringerung
der Höhe und Aktivität des epithelialen Natriumkanals führen, was die Herausbildung einer Hyperpermeabilität begünstigt. Es ist auch bekannt, dass bei diesen viralen Erkrankungen der Lunge die häufig verwendeten beta2-adrenerge Agonisten keine Wirkungen zeigen.

Insgesamt ist also bekannt, dass mikrobielle Toxine zu einem erhöhten Spiegel von Reaktiven Sauerstoffmolekülen in Endothel- und Epithelzellen führen. Dadurch kommt es zur Phosphorylierung der Myosin Light Chain, was wiederum zu einer Störung der Zell-Zell Interaktion und zur Ausbildung einer Hyperpermeabilität führt.

Mikrobielle Toxine, Reaktive Sauerstoffmoleküle, sowie virale Proteine führen zu einer Aktivierung von Protein Kinase C Isoenzymen. Aktivierung der Protein Kinase C führt dann zu einer Herabsetzung der Expression des epithelialen Natriumkanals (E-NaC) und Hemmung seiner Aktivität. Auch diese Mechanismen führen zur Herausbildung einer Hyperpermeabilität im Endothel und Epithel.

Eine Hyperpermeabilität von Geweben der Lunge ist wesentlicher Bestandteil verschiedener Erkrankungen der Lunge, z.B. der Akuten Lungenverletzung, des Akuten Respiratorischen Distress Syndrom (ARDS), der Lungenentzündung (Pneumonie). Derzeit gibt es keine Standardtherapie zur Behandlung einer Hyperpermeabilität des Endothels und des Epithels.

DE 600 04 340 T2 beschreibt bestimmte Tyrosinkinase-Hemmer (substituierte 1,4-Dihydroindeno[1,2-C]pyrazole) zur Verhinderung vaskulärer Hyperpermeabilität und der damit einhergehenden Ödeme.

US 2003/0185791 A1, EP 2 009 023 A1, WO 2006/013183 A1, EP 1 264 559 A1 und Marquardt et al. (J. Pept. Sci. 13 (2007): 803-810) offenbaren TNF-abgeleitete Peptide zur Behandlung von Ödemen.

Es ist daher Aufgabe der vorliegenden Erfindung, Mittel und Verfahren zur Verfügung zu stellen, mit denen Erkrankungen, bei denen die Verhinderung einer Hyperpermeabilität von Epithelzellen und Endothelzellen eine wesentliche Rolle bei der Behandlung spielt, insbesondere Lungenerkrankungen, wie akute Lungenverletzungen, ARDS oder virale Lungenkrankheiten, verhindert oder behandelt werden können.

Insbesondere soll mit der Erfindung ein biologisch wirksames Molekül zur Vorbeugung und Behandlung der Hyperpermeabilität des Endothels und Epithels und zur Vorbeugung und Behandlung des Akuten Lungenschadens und den Folgen der Pneumonie bereitgestellt werden.

Demgemäß betrifft die vorliegende Erfindung ein Peptid, welches aus 7-17 benachbarten Aminosäuren besteht und das Hexamer TX₁EX₂X₃E umfasst, wobei X₁, X₂ und X₃ jede natürliche oder nicht natürliche Aminosäure sein kann, wobei das Peptid keine TNF-Rezeptor-Bindungsaktivität aufweist und zyklisiert ist, zur Anwendung zur Verhinderung von Ödemen durch Verringerung der Hyperpermeabilität, beruhend auf Verletzungen der Endothel- und Epithelschichten.

Vorzugsweise betrifft die vorliegende Erfindung ein Peptid, welches aus 7-17 benachbarten Aminosäuren besteht und das Hexamer TPEGAE (SEQ ID No. 4) umfasst, wobei das Peptid keine TNF-Rezeptor-Bindungsaktivität aufweist und zyklisiert ist, zur Anwendung zur Verhinderung von Ödemen durch Verringerung der Hyperpermeabilität, beruhend auf Verletzungen der Endothel- und Epithelschichten .

Eine besonders bevorzugte Ausführungsform der vorliegenden Erfindung betrifft ein zyklisiertes Peptid, bestehend aus einer Sequenz aufeinanderfolgender Aminosäuren, ausgewählt aus der Gruppe bestehend aus
- QRETPEGAEAKPWY (SEQ ID No. 5)
- PKDTPEGAELKPWY (SEQ ID No. 6)
- CGQRETPEGAEAKPWYC (SEQ ID No. 1) und
- CGPKDTPEGAELKPWYC (SEQ ID No. 7)
und Fragmenten von mindestens 7 Aminosäuren davon, welche Fragmente des Hexamer TPEGAE aufweisen, zur Herstellung eines Medikaments zur Anwendung zur Verhinderung von Ödemen durch Verringerung der Hyperpermeabilität, beruhend auf Verletzungen der Endothel- und Epithelschichten .

Die erfindungsgemäßen Peptide werden bevorzugter Weise zur Anwendung zur Verhinderung von Ödemen bei Pneumonie, akuter Lungenverletzung, Akutem Respiratorischen Distress-Syndrom (ARDS) oder bakteriellen oder viralen Lungenkrankheiten, insbesondere Infektionen mit Listeria monocytogenes, Streptococcus pneumoniae, Influenza-Viren, SARS oder RSV, verwendet. Die Ursache der Lungenentzündung (Pneumonie), die erfindungsgemäß behandelt oder verhindert werden kann, ist unabhängig von der Ursache der Lungenentzündung und unabhängig davon, ob es sich um eine akute oder chronische Entzündung handelt. Demgemäß können erfindungsgemäß vorzugsweise Pneumonien, die durch eine Infektion mit Bakterien, Viren, Mykoplasmen, Protozoen, Würmer oder Pilzen verursacht sind, behandelt werden, jedoch auch toxisch (z.B. durch Inhalation giftiger Stoffe), immunologisch oder durch Strahlen (z.B. Röntgenstrahlung, Bestrahlungstherapie bei Krebspatienten) verursachte Lungenentzündungen. Gerade bei den durch Inhalierung giftiger Stoffe oder Bestrahlung verursachten Lungenentzündungen ist der präventive Aspekt der vorliegenden Erfindung besonders wesentlich, jedoch auch bei bettlägrigen Personen, insbesondere älteren Menschen, oder bei immunsupprimierten Personen, wie HIV-Patienten oder Transplantationspatienten. Insbesondere kann erfindungsgemäß die Lungenentzündung zu einem Zeitpunkt bekämpft oder verhindert werden, zu dem auf dem Rönt-genbild noch keine Schäden erkennbar sind.

Erreger primärer Lungenentzündungen sind meistens Pneumokokken, Staphylokokken, Haemophilus influenzae, Mykoplasmen, Chlamydien, Legionellen (Legionella pneumophila) und Viren wie das Grippevirus, Adenovirus und Parainfluenzaviren. Das Erregerspektrum verschiebt sich bei sekundären Pneumonien zu Herpesviren (CMV, HSV), Pilzen, Pneumocystis jirovecii, Protozoen (Toxoplasmose) sowie anaeroben Bakterien. Insbesondere Lungenentzündungen, die durch diese Erreger verursacht werden, sind erfindungsgemäß besonders bevorzugt behandelbar bzw. (insbesondere im Hinblick auf sekundäre Pneumonien) verhinderbar.

Die erfindungsgemäßen Peptide sind beispielsweise aus dem europäischen Patent EP-1 264 599-B1 bekannt und wurden im Stand der Technik zur Behandlung von Flüssigkeitsansammlungen (Lungenödemen) und insbesondere zur Reabsorption dieser Flüssigkeitsansammlungen vorgeschlagen, wobei die Ödemflüssigkeit aus den Alveolen des Lungengewebes in die Kapillaren zurückgeführt, also aus den Alveolen herausgepumpt, wird.

Es hat sich erfindungsgemäß völlig überraschend gezeigt, dass diese Peptide auch den entgegengerichteten Flüssigkeitsstrom über das Endothel der Kapillaren in das Epithel der Lunge beeinflussen, jedoch auf gegenteilige Weise: Während bei der Ödembehandlung das Heraustransportieren der Flüssigkeit offene und voll aktive Pumpmechanismen erfordert, wird erfindungsgemäß das Übertreten der Flüssigkeit in die Alveolen gestoppt; der Zustrom wird daher von vornherein unterbunden. Die Aktivierung der Ödemresorption gemäß EP 1 264 599 B1 durch die erfindungsgemäßen Peptide scheint daher einem ganz anderen - in die Gegenrichtung verlaufenden und regulierenden - Mechanismus zugrunde zu liegen als die erfindungsgemäße Verringerung der Hyperpermeabilität beruhend auf Verletzungen der Endothel- und Epithelschichten, wodurch Ödeme durch Vermeidung des Flüssigkeitsübertrittes in die Alveolen sogar verhindert werden. Demgemäß erschließen sich mit der vorliegenden Erfindung - neben der Ödem-Behandlung aus der EP 1 264 599 B1 (die erst in einem späten Stadium des Krankheitsverlaufes angezeigt ist) - völlig neue und überraschende Indikationen für die erfindungsgemäßen Peptide.

Die vorliegende Erfindung beruht demgemäß auch auf dem im Zuge der Arbeiten zur Erfindung aufgefundenen Umstand, dass die erfindungsgemäß verwendeten Peptide, wie sie in der EP 1 264 599 B1 definiert worden sind, einen Einfluss auf die Wirkungen von To-xinen, Reaktiven Sauerstoffmolekülen, auf die Aktivierung der Protein Kinase C, Phosphorylierung der Myosin Light Chain, und Expression des epithelialen Natriumkanals haben. Dies war aufgrund des vorhandenen Wissens über diese Peptide nicht zu erwarten.

Ein ganz besonders bevorzugtes Peptid zur Anwendung gemäß der vorliegenden Erfindung besteht aus der Aminosäuresequenz CGQRETPEGAEAKPWYC und ist über die C-Reste (an Position 1 und 17) zyklisiert.

Die Zyklisierung der erfindungsgemäßen Peptide kann entweder über eine direkte Zyklisierung über eine Disulfidbrücke zwischen den beiden C-Resten am N- und C-Terminus erreicht werden oder aber indem das Peptid über beide Cysteine an eine Trägersubstanz gekoppelt wird. Dabei werden in den erfindungsgemäßen Peptiden die Cysteinreste vorzugsweise am Anfang und am Ende des Moleküls vorgesehen. Auch andere funktioneile Gruppen, die eine Zyklisierung des Peptids erreichen, können eingesetzt werden, z.B. indem eine Säuregruppe mit einem Amin oder Alkohol zu einem Amid- oder Ester-Ringschluss führt (hierbei können z.B. die Aminosäuren Asparaginsäure und Glutaminsäure mit Serin, Threonin, Tyrosin, Asparagin, Glutamin oder Lysin, vorzugsweise intramolekular zyklisiert werden). Weitere bevorzugte erfindungsgemäße Peptide sind daher beispielsweise CGQKETPEGAEAKPWYC (SEQ ID No. 8), CGQRETPEGAEARPWYC (SEQ ID No. 9), CGQRETPEGAEAKPC (SEQ ID No. 10), CQRETPEGAEAKPWYC (SEQ ID No. 11) oder CGQRETPEGAEAKFWYC (SEQ ID No. 12).

Als Trägersubstanzen kommen alle gängigen pharmazeutisch verwendbaren Substanzen in Frage, die in der Lage sind, z.B. mit den SH-Gruppen der Cysteine eine kovalente Bindung einzugehen, wobei gängige Trägerproteine, wie Keyhole limpet hemocyanin (KLH), Tetanus-Toxin etc. sich besonders eignen. Auch können am Träger benachbarte bifunktionelle Reste vorgesehen werden (z.B. Säuregruppe neben Amin- oder Alkoholgruppe). In diesem Zusammenhang ist wichtig, dass mit "Zyklisierung" sowohl der intramolekulare Ringschluss als auch die Einbindung eines Trägers umfasst (von dem das gebundene Peptid hervorragt (indem der N- und der C-Terminus des Peptids an den Träger gebunden ist)), wobei das derart zyklisierte Peptid die zyklische Raumstruktur zeigt und entsprechend stabilisiert ist.

Die erfindungsgemäßen Peptide können bevorzugter Weise zum Schutz von Epithelzellen und Endothelzellen vor Hyperpermeabilität, ausgelöst durch Reaktive Sauerstoffmoleküle oder durch bakterielle Toxine, verwendet werden.

Die erfindungsgemäßen Peptide können auch zur Hemmung der Phosphorylierung der Myosin Light Chain, zur Hemmung der Aktivierung der Protein Kinase C oder zur Erhöhung der Expression des epithelia-len Natriumkanals verwendet werden.

Dabei können die erfindungsgemäßen Peptide zur Verhinderung von Ödemen durch Verringerung einer Hyperpermeabilität, ausgelöst durch Reaktive Sauerstoffmoleküle, mikrobielle Toxine, Gram-positive Mikroorganismen oder durch pulmonalen Virenbefall, verwendet werden.

Gemäß einem weiteren Aspekt betrifft die vorliegende Erfindung eine pharmazeutische Zusammensetzung, enthaltend ein erfindungsgemäßes Peptid (oder eine Mischung verschiedener erfindungsgemäßer Peptide) und einen pharmazeutischen Träger. Diese pharmazeutische Zusammensetzung wird erfindungsgemäß zur Verhinderung und Behandlung von Hyperpermeabilität, wie oben beschrieben, eingesetzt, insbesondere zur Verhinderung und Behandlung von Pneumonie, akuter Lungenverletzung, Akutem Respiratorischen Distress-Syndrom (ARDS) oder viralen Lungenkrankheiten, insbesondere Infektionen mit Listeria monocytogenes, Streptococcus pneumoniae, SARS, RSV, oder Influenza-Viren, insbesondere Influenza A Viren. Der Ausdruck "eine pharmazeutische Zusammensetzung" bezieht sich auf jede Zusammensetzung, die ein Peptid, wie oben definiert, umfasst, welche die hierin beschriebenen Zustände verhindert, verbessert oder heilt. Insbesondere bezieht sich der Ausdruck "eine pharmazeutische Zusammensetzung" auf eine Zusammensetzung, die ein Peptid, wie oben beschrieben, und einen pharmazeutisch akzeptablen Träger oder Exzipienten (beide Ausdrücke können austauschbar verwendet werden) aufweist. Geeignete Träger oder Exzipienten, die dem Fachmann bekannt sind, sind Kochsalzlösung, Ringer-Lösung, Dextrose-Lösung, Hank-Lösung, fixierte Öle, Ethyloleat, 5% Dextrose in Kochsalzlösung, Substanzen, die die Isotonie und die chemische Stabilität verbessern, Puffer und Konservierungsmittel. Andere geeignete Träger inkludieren jeden Träger, der nicht selbst die Produktion von Antikörpern, die für das die Zusammensetzung erhaltende Individuum schädlich sind, induziert, wie Proteine, Polysaccharide, Polymilchsäuren, Polyglykolsäuren, polymere Aminosäuren und Aminosäure-Copolymere. Dabei kann das erfindungsgemäße Peptid auch durch direkte kovalente Bindung an diese Träger zyklisiert werden. Diese pharmazeutische Zusammensetzung kann (als Medikament) mit jedem im Wissen des Fachmanns liegenden geeigneten Verfahren verabreicht werden. Der bevorzugte Verabreichungsweg ist parenteral, insbesondere durch Inhalation (durch Aerosole) oder intravenöse Gabe. Bei parenteraler Verabreichung wird das Medikament dieser Erfindung in injizierbarer Dosiseinheitsform, wie als Lösung, Suspension oder Emulsion, in Verbindung mit den oben definierten pharmazeutisch akzeptablen Exzipienten formuliert. Die Dosierung und Verabreichungsart hängt jedoch vom Individuum ab. Im Allgemeinen wird das Medikament so verabreicht, dass das Peptid der vorliegenden Erfindung in einer Dosis von zwischen 1 µg/kg und 10 mg/kg, mehr bevorzugt zwischen 10 ug/kg und 5 mg/kg, am meisten bevorzugt zwischen 0,1 und 2 mg/kg, gegeben wird. Vorzugsweise wird es als Bolus-Dosis gegeben. Es kann auch eine kontinuierliche Infusion verwendet werden. In diesem Fall kann das Medikament in einer Dosis von zwischen 5 und 20 µg/kg/Minute, mehr bevorzugt zwischen 7 und 15 µg/kg/Minute, infundiert werden.

Gemäß vorliegender Erfindung hat ein besonders bevorzugtes erfindungsgemäßes Peptid folgende Aminosäuresequenz: SEQ ID:NO:1 (NH2)Cys-Gly-Gln-Arg-Glu-Thr-Pro-Glu-Gly-Ala-Glu-Ala-Lys-Pro-Trp-Tyr-Cys(COOH).

Die Bestimmung der Konzentration an Reaktiven Sauerstoffmolekülen in kultivierten Endothelzellen der Lunge ergab, dass es bei einer Kultur der Endothelzellen unter normalem Sauerstoffgehalt von 21% (Nomoxia Gasgemisch) nur zu einer geringen Bildung von Reaktiven Sauerstoffmolekülen kommt. Unter Sauerstoffmangel (0,1% Sauerstoff, Hypoxia Gasgemisch) kommt es jedoch zu einer 3-fach höheren Bildung von Reaktiven Sauerstoffmolekülen. Wird jedoch ein erfindungsgemäßes Peptid, insbesondere das Peptid SEQ ID No. 1, zu Endothelzellen, welche unter Sauerstoffarmut (Sauerstoffgehalt 0,1%, Hypoxia Gasgemisch) kultiviert werden, hinzugegeben, so werden überraschenderweise keine Reaktiven Sauerstoffmoleküle durch die Endothelzellen gebildet.

Weitere Untersuchungen bestimmten den elektrischen Widerstand von Zellschichten von menschlichen Endothel- und Epithelzellen mittels elektrischer Zell-Substrat Impedanzanalyse, vor, während und nach der Zugabe der mikrobiellen Toxine Pneumolysin und Listeriolysin. Die Untersuchungen zeigten, dass durch eine Zugabe von 125 ng/ml und 250 ng/ml Listeriolysin zu kultivierten menschlichen Endothelzellen die Herausbildung einer Hyperpermeabilität initiiert wird. Dieser Vorgang wurde durch eine Toxinkonzentration von 250 ng/ml Listeriolysin noch verstärkt. Auch die Zugabe von 62,5 ng/ml Pneumolysin zu kultivierten menschlichen Endothelzellen führt zur Ausprägung einer Hyperpermeabilität. Dieser Vorgang wurde durch eine Toxinkonzentration von 125 ng/ml Pneumolysin noch verstärkt. Überraschenderweise wurde jedoch gefunden, dass durch die Zugabe jedoch von erfindungsgemäßem Peptid, insbesondere 50 µg/ml des Peptides SEQ ID No. 1, sowohl die Pneumolysin-induzierte und die Listeriolysin induzierte Hyperpermeabilität inhibiert wird.

Weitere Untersuchungen zeigten, dass auch in menschlichen Epithelzellen durch mikrobielle Toxine eine Hyperpermeabilität induziert werden kann. So führt die Inkubation von menschlichen Epithelzellen mit 1 µg/ml Listeriolysin zu einer deutlichen Hyperpermeabilität. Überraschenderweise wurde jedoch gefunden, dass die Hyperpermeabilität durch eine Zugabe von erfindungsgemäßem Peptid, insbesondere 50 µg/ml Peptid SEQ ID No. 1, inhibiert wird.

Weitere Untersuchungen zeigten, dass eine Zugabe des Toxin 125 ng/ml Listeriolysin zu menschlichen endothelialen Lungenzellen zu einer Zunahme des Gehalts an phosphorylierter Myosin Light Chain führt. Dieser Effekt wird durch eine Toxinkonzentration von 250 ng/ml Listeriolysin noch verstärkt. Auch eine Zugabe von 62,5 ng/ml des Toxins Pneumolysin zu menschlichen endothelialen Lungenzellen führte zu einer Zunahme des relativen Gehalts an phosphorylierter Myosin Light Chain. Dieser Effekt wurde durch eine Toxinkonzentration von 125 ng/ml Pneumolysin noch verstärkt. Überraschenderweise wurde jedoch gefunden, dass eine Zugabe von erfindungsgemäßem Peptid, insbesondere 50 µg/ml des Peptides SEQ ID No. 1, die durch die Toxine Listeriolysin und Pneumolysin hervorgerufene Phosphorylierung der Myosin Light Chain hemmt.

Weitere Untersuchungen ergaben, dass durch die intratracheale Applikation von Toxinen in Mäuse eine Hyperpermeabilität der Lungen von Mäusen ausgelöst wird, was dadurch nachgewiesen wurde, dass der Farbstoff Evans Blue aus den Blutgefäßen in das Lungengewebe übertritt. Überraschenderweise wurde jedoch gefunden, dass es durch die intratracheale Applikation eines erfindungsgemäßen Peptids, insbesondere von 50 µg Peptid SEQ ID No. 1, zu einer Hemmung der durch das Toxin-bedingten Hyperpermeabilität kommt.

Weitere Untersuchungen zeigten, dass durch die Auslösung der Hyperpermeabilität in Lungen von Mäusen, ausgelöst durch intratracheale Applikation von Toxin, z.B. 250 ng Pneumolysin, es zu einer erhöhten Anzahl von Leukozyten in der bronchioalvo-laren Flüssigkeit kommt. Überraschenderweise wurde jedoch gefunden, dass durch die intratracheale Applikation von erfindungsgemäßem Peptid, insbesondere 50 µg Peptid SEQ ID No. 1, die Toxin-bedingte Herausbildung der Hyperpermeabilität gehemmt wird, und deutlich weniger Leukozyten in der bronchioalvolaren Flüssigkeit von Lungen aus Mäusen vorkommen.

Weitere Untersuchungen ergaben, dass bakterielles Toxin zu einer wesentlichen Erhöhung des Gehalts an aktivierter Protein Kinase C alpha in menschlichen Endothelzellen der Lunge führt. Überraschenderweise wurde jedoch gefunden, dass eine Zugabe von erfindungsgemäßem Peptid, insbesondere von Peptid SEQ ID No. 1, diese Toxin-vermittelte Wirkung inhibiert und somit zur Erhöhung der Expression des epithelialen Natriumkanals führt. Überraschend war auch zu finden, dass eine Zugabe des erfindungsgemäßen Peptids, insbesondere des Peptides SEQ ID No. 1, zu menschlichen epithelialen Zellen zu einer wesentlichen Erhöhung der Expression des epithelialen Natriumkanals (ENaC) führt.
Die Erfindung wird anhand der nachfolgenden Beispiele und der Zeichnungsfiguren, auf die sie aber selbstverständlich nicht beschränkt ist, näher erläutert.

Fig. 1A zeigt das HPLC Chromatogramm des Proteins mit der Aminosäuresequenz SEQ ID No: 1. Einheiten: y-Achse "Absorption in mAU"; x-Achse "Zeit in Minuten".
Fig. 1B zeigt das HPLC Chromatogramm des Proteins mit der Aminosäuresequenz SEQ ID No: 2. Einheiten: y-Achse "Absorption in mAU"; x-Achse "Zeit in Minuten".
Fig. 1C zeigt das HPLC Chromatogramm des Proteins mit der Aminosäuresequenz SEQ ID No: 3. Einheiten: y-Achse "Absorption in mAU"; x-Achse "Zeit in Minuten".
Figur 2A zeigt die paramagnetischen Elektronenresonanz (EPR), Spektren von Endothelzellen, welche entweder bei 21% Sauerstoff (Normoxia Gasgemisch) oder bei 0,1% Sauerstoff (Hypoxia Gasgemisch) mit und ohne Zugabe des Peptides SEQ ID No. 1 bzw. Peptid SEQ ID No. 3 kultiviert wurden.
Figur 2B zeigt den relativen Gehalt an Reaktiven Sauerstoffmolekülen (Superoxid) in Endothelzellen, welche entweder bei 21% Sauerstoff (Normoxia Gasgemisch) oder bei 0,1% Sauerstoff (Hypoxia Gasgemisch) mit und ohne Zugabe des Peptides SEQ ID No. 1, oder bei 0,1% Sauerstoff (Hypoxia Gasgemisch) mit und ohne Zugabe des Peptides SEQ ID No. 3 kultiviert wurden.
Figur 3A zeigt den Verlauf des elektrischen Widerstandes von menschlichen Epithelzellen der Lunge ohne Zugabe von Toxin Listeriolysin, sowie nach Zugabe von 125 ng/ml Listeriolysin (125 ng/ml LLO) und nach Zugabe von 500 ng/ml Listeriolysin (500 ng/ml LLO).
Figur 3B zeigt den Verlauf des elektrischen Widerstandes von menschlichen Epithelzellen der Lunge ohne Zugabe von Toxin Pneumolysin, sowie nach Zugabe von 62,5 ng/ml Pneumolysin (62,5 ng/ml PLY) und nach Zugabe von 250 ng/ml Pneumolysin (250 ng/ml PLY) .
Figur 3C zeigt den Verlauf des elektrischen Widerstandes von menschlichen Epithelzellen der Lunge ohne Zugabe von Toxin Pneumolysin / Peptid SEQ ID No. 1 (Kontrolle), sowie nach Zugabe von 125 ng/ml Pneumolysin (125 ng/ml PLY) und nach Zugabe von 125 ng/ml Pneumolysin / 50 µg/ml Peptid SEQ ID No. 1 (125 ng/ml PLY / 50 µg/ml Peptid SEQ ID No. 1).
Figur 3D zeigt den Verlauf des elektrischen Widerstandes von menschlichen Epithelzellen der Lunge ohne Zugabe von Toxin Listeriolysin / Peptid SEQ ID No. 1 (Kontrolle), sowie nach Zugabe von 500 ng/ml Listeriolysin (500 ng/ml LLO) und nach Zugabe von 500 ng/ml Listeriolysin / 50 µg/ml Peptid SEQ ID No. 1 (500 ng/ml LLO / 50 µg/ml Peptid SEQ ID No. 1).
Figur 3E zeigt den Verlauf des elektrischen Widerstandes von menschlichen Endothelzellen der Lunge ohne Zugabe von Toxin Listeriolysin / Peptid SEQ ID No. 1 (Kontrolle), sowie nach Zugabe von 1 µg/ml Listeriolysin (1 µg/ml LLO) und nach Zugabe von 1 µg/ml Listeriolysin / 50 µg/ml Peptid SEQ ID No. 1 (1 µg/ml LLO / 50 µg/ml Peptid SEQ ID No. 1).
Figur 4A zeigt den relativen Gehalt von phosphorylierter Myosin Light Chain in menschlichen Endothelzellen der Lunge in Abhängigkeit von der Konzentration des Toxin Listeriolysin (125 ng/ml LLO, 250 ng/ml LLO, 500 ng/ml LLO).
Figur 4B zeigt den relativen Gehalt von phosphorylierter Myosin Light Chain in menschlichen Endothelzellen der Lunge in Abhängigkeit von der Konzentration des Toxin Pneumolysin (62,5 ng/ ml PLY, 125 ng/ml PLY, 250 ng/ml PLY).
Figur 4C zeigt den relativen Gehalt von phosphorylierter Myosin Light Chain in menschlichen Endothelzellen der Lunge in Abhängigkeit von der Zugabe von 50 µg/ml Peptid SEQ ID No. 1, 250 ng/ml Toxin Listeriolysin (LLO), 50 µg/ml Peptid SEQ ID No. 1 / 250 ng/ml Toxin Listeriolysin (LLO), 50 µg/ml Peptid SEQ ID No. 3 / 250 ng/ml Toxin Listeriolysin (LLO).
Figur 4D zeigt den relativen Gehalt von phosphorylierter Myosin Light Chain in menschlichen Endothelzellen der Lunge in Abhängigkeit von der Zugabe von 50 µg/ml Peptid SEQ ID No. 1, 125 ng/ml Toxin Pneumolysin (PLY), 50 µg/ml Peptid SEQ ID No. 1 / 125 ng/ml Toxin Pneumolysin (PLY), 50 µg/ml Peptid SEQ ID No. 3 / 125 ng/ml Toxin Pneumolysin (PLY).
Figur 5A zeigt den Gehalt Evans Blau Farbstoff im Lungengewebe von Mäusen 5,5 Stunden nach der intratrachealen Verabreichung des Toxins Pneumolysin mit den Dosen 250 ng Pneumolysin pro Maus (250 ng PLY) und 500 ng Pneumolysin pro Maus (500 ng PLY) .
Figur 5B zeigt den Gehalt Evans Blau Farbstoff im Lungengewebe von Mäusen 5,5 Stunden nach der intratrachealen Verabreichung von 250 ng Toxin Pneumolysin pro Maus, sowie nach der intratrachealen Verabreicherung von 250 ng Toxin Pneumolysin und 50 ug Peptid SEQ ID No. 1 pro Maus.
Figur 5C zeigt den Gehalt von Leukozyten in der brochioalvolaren Flüssigkeit der Lungen von Mäusen 5,5 Stunden nach der intratrachealen Verabreichung von 250 ng Toxin Pneumolysin pro Maus, sowie nach der intratrachealen Verabreichung von 250 ng Toxin Pneumolysin und 50 µg Peptid SEQ ID No. 1 pro Maus.
Figur 6 gibt den Gehalt an aktivierter Protein Kinase C alpha im Verhältnis zum Gesamtgehalt an Protein Kinase C alpha an, in Abhängigkeit von der Inkubation menschlicher endothialer Lungenzellen mit 250 ng/ml Toxin Pneumolysin (250 ng/ml PLY) und der Mischung aus 250 ng/ml Toxin Pneumolysin und 50 µg/ml Peptide SEQ ID No. 1 (250 ng/ml PLY/50 µg/ml Peptid SEQ ID No. 1).
Figur 7 zeigt die Expression des epithelialen Natriumkanals (ENaC) in menschlichen epithelialen Lungenzellen im Vergleich zu Zellkulturbedingungen ohne und nach Zugabe von 50 ug/ml Peptid SEQ ID No. 1, sowie nach Zugabe von 50 ug/ml Peptid SEQ ID No. 3. Der Gehalt an mRNS für ENaC wurde mittels "Real-Time PCR" bestimmt.
Figur 8 zeigt die Veränderung des Körpergewichts der Versuchtiere bei einer viralen Lungenentzündung (Gruppe 1: negative Kontrolle (PBS); Gruppe 2: positive Kontrolle (Influenza A per nasal); Gruppe 3: Influenza A per nasal + 10µg Peptid SEQ.ID.NO. 1 intratrachial);
Figur 9 zeigt die Veränderung der Körpertemperatur von Versuchstieren dieser Gruppen 1 bis 3;
Figur 10 zeigt die Überlebensrate von Versuchstieren dieser Gruppen 1 bis 3.

### Beispiele

Beispiel 1A: Synthese eines Peptides mit der Aminosäuresequenz SEQ ID NO: 1

Ein Peptid mit der Aminosäuresequenz SEQ ID NO: 1 wurde mittels Fmoc-Festphasensynthese vollautomatisch in folgenden Schritten synthetisiert:

| Schritt | Prozess | Produkt |
|---|---|---|
| 1 | Kopplung der Aminosäuren | Peptid gebunden an die Festphase |
| 2 | Abspaltung von der Festphase | Peptid in Lösung |
| 3 | Reinigung | Gereinigtes Peptid als TFA-Salz |
| 4 | Reinigung / Salz Austausch | Gereinigtes Peptid als Acetatsalz |
| 5 | Analytische Untersuchung | Gereinigtes Peptid |

Anschließend wurde das Peptid SEQ ID No. 1 durch oxidative Herausbildung einer Disulfidbrücke zwischen den Seitenketten der Aminosäuren Cystein (Position 1) und Cystein (Position 17) zyklisiert.

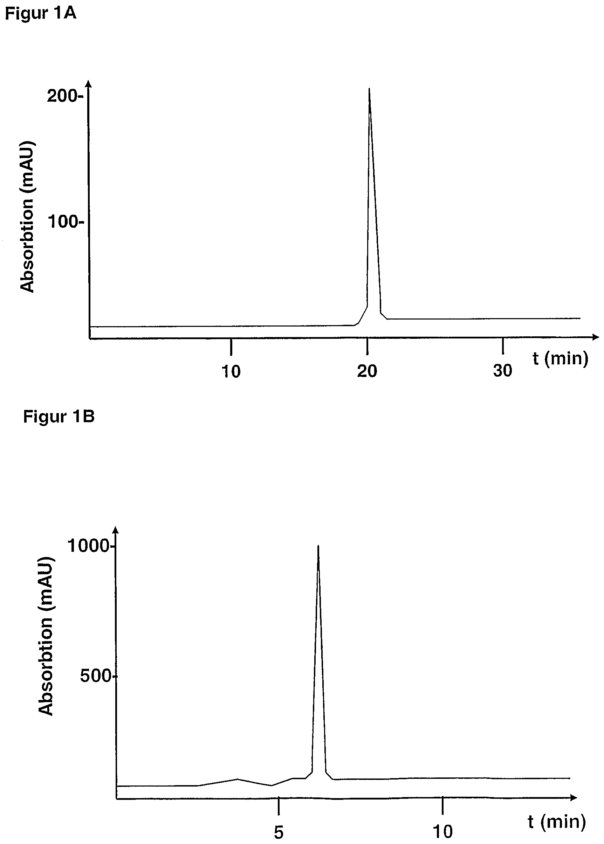

Anschließend wurde das Peptid mittels Reverser HPLC untersucht, wobei das Ergebnis wie in Fig. 1A gezeigt erhalten wurde. Die Reinheit des Peptides SEQ ID No. 1 war größer als 95%.

### Beispiel 1B: Synthese eines Peptides mit der Aminosäuresequenz SEQ ID No. 2

worin eine Amidbindung zwischen der Aminogruppe der Seitenkette des Lysins Lys (l) und der Carboxylgruppe der Seitenkette der Glutaminsäure Glu (19) ausgebildet ist.

Ein Peptid mit der Aminosäuresequenz SEQ ID No. 2 wurde mittels Fmoc-Festphasensynthese vollautomatisch in folgenden Schritten synthetisiert:

| Schritt | Prozess | Produkt |
|---|---|---|
| 1 | Kopplung der Aminosäuren | Peptid gebunden an die Festphase |
| 2 | Abspaltung von der Festphase | Peptid in Lösung |
| 3 | Reinigung | Gereinigtes Peptid als TFA-Salz |
| 4 | Reinigung / Salz Austausch / oxidative Zyklisierung | Gereinigtes Peptid als Acetatsalz |
| 5 | Analytische Untersuchung | Gereinigtes Peptid |

Die Zyklisierung erfolgte durch die Verbindung der epsilon-Aminogruppe des Lysins (Position 1) mit der gamma-Karboxylgruppe der Glutaminsäure (Position 19) unter Bildung einer Amidbindung. Dies erfolgt zum Beispiel dadurch, dass die gamma-Karboxylgruppe der Glutamingruppe mittels Dicyclohexylcarbodiimid (DHC) in einen Aktivester überführt wird, welcher anschließend spontan mit der epsilon-Aminogruppe des Lysins unter Ausbildung eines Ringschlusses im Peptid reagiert.

Anschließend wurde das Peptid mittels Reverser HPLC untersucht, wobei das Ergebnis wie in Fig. 1B gezeigt, erhalten wurde. Die Reinheit des Peptides SEQ ID No. 2 war größer als 95%.

### Beispiel 1C: Synthese eines Peptides mit der Aminosäuresequenz SEQ ID No. 3

Ein Peptid mit der Aminosäuresequenz SEQ ID No. 3 wurde mittels Fmoc-Festphasensynthese vollautomatisch in folgenden Schritten synthetisiert:

| Schritt | Prozess | Produkt |
|---|---|---|
| 1 | Kopplung der Aminosäuren | Peptid gebunden an die Festphase |
| 2 | Abspaltung von der Festphase | Peptid in Lösung |
| 3 | Reinigung | Gereinigtes Peptid als TFA-Salz |
| 4 | Reinigung / Salz Austausch | Gereinigtes Peptid als Acetatsalz |
| 5 | Analytische Untersuchung | Gereinigtes Peptid |

Anschließend wurde das Peptid SEQ ID No. 3 durch oxidative Herausbildung einer Disulfidbrücke zwischen den Seitenketten der Aminosäuren Cystein (Position 1) und Cystein (Position 17) zyklisiert.

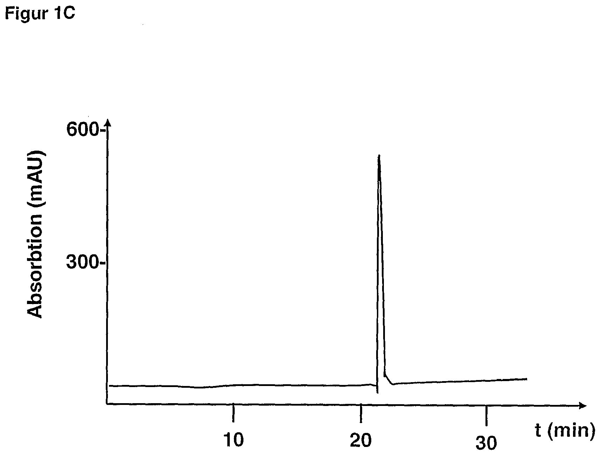

Anschließend wurde das Peptid mittels Reverser HPLC untersucht, wobei das Ergebnis wie in Fig. 1C gezeigt erhalten wurde. Die Reinheit des Peptides SEQ ID No. 3 war größer als 95%. Der Unterschied zwischen Peptid SEQ ID No. 3 zum Peptid SEQ ID No. 1 besteht darin, dass die Aminosäuren Thr(6), Glu(8), Glu(11) aus Peptid SEQ ID No. 1 in Ala(6), Ala(8) und Ala(11) im Peptid SEQ ID No. 3 vorliegen.

### Beispiel 2: Einfluss des Peptides SEQ ID No. 1 auf Reaktive Sauerstoffmoleküle

### Zellkultur von Endothelzellen

Die Zellkultur von Endothelzellen erfolgte mit Zusatz und ohne Zusatz von 50 µg/ml Peptid SEQ ID No. 1 bzw. mit Zusatz und ohne Zusatz von 50 µg/ml Peptid SEQ ID No. 3.

Arterielle Endothelzellen wurden zur Erzeugung von Reaktiven Sauerstoffmolekülen in einem sauerstoffarmen Gasgemisch aus 0,1% Sauerstoff, 5% Kohlendioxid und 94,9% Stickstoff kultiviert (Hypoxia Gasgemisch). In Kontrollexperimenten betrugen die Gaskonzentrationen 21% Sauerstoff, 5% Kohlendioxid und 74% Stickstoff (Normoxia Gasgemisch).

Nach 90 Minuten unter den sauerstoffarmen Bedingungen wurden die Endothelzellen weitere 30 Minuten mit 21% Sauerstoff kultiviert. Danach wurde zu den Zellen 20 µl einer Lösung bestehend aus 20 uM 1-Hydroxy-3-methoxycarbonyl-2,2,5,5-tetramethylpyrrolidine HCl (CHM), 20 µM DPBS, 25 µM Desferrioxa-min und 5 µM Diethyldithiocarbamat, sowie 2 µl DMSO, zugegeben. Trypsinisierung der Zellen

Nach der Zellkultur wurden die Zellen in laborüblicher Weise durch die Zugabe einer Trypsinlösung vereinzelt. Die Endothelzellen wurden gewaschen und in 35 µl einer Lösung bestehend aus DPBS und 25 µM Desferrioxamin und 5 µM Diethyldithiocarbamat suspendiert.

### Messung der Paramagnetischen Elektronenresonanz (EPR)

Die Bestimmung der Paramagnetischen Elektronenresonanz (EPR), auch Elektronenspinresonanz genannt, dient zur Untersuchung von paramagnetischen Stoffen, z.B. zur Detektion von ungepaarten Elektronen in Reaktiven Sauerstoffmolekülen (Radikale des Sauerstoffs).

Dazu wurden die zuvor behandelten Zellen in 50 µl Kapillaren gegeben und in einem MiniScope MS200 ESR der Firma Magnettech (Berlin, Deutschland) bei 40 mW Mikrowellen, 3000 mG Modulationsamplitude, 100 kHz Modulationsfrequenz untersucht.

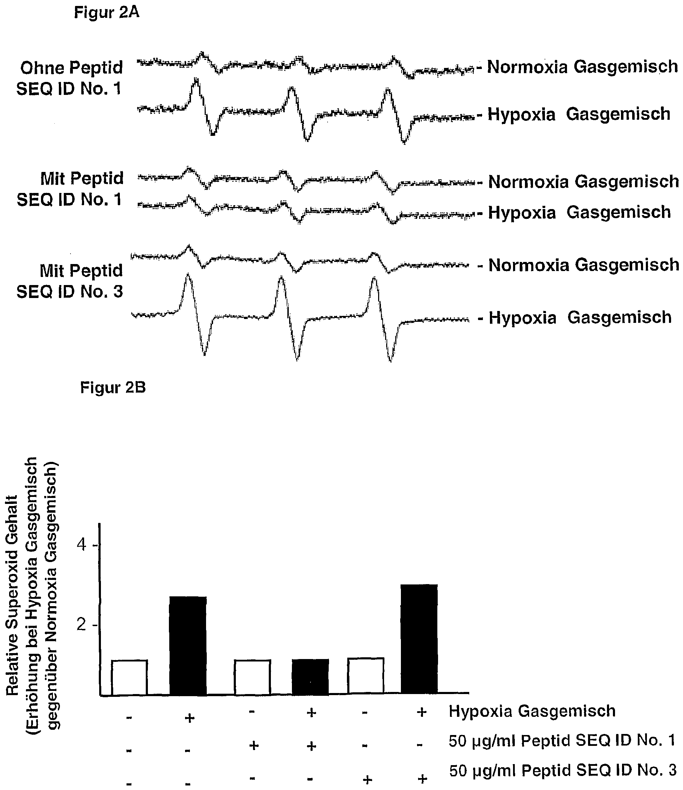

Wie die Figuren 2A und 2B zeigen, kommt es bei normaler Sauerstoff konzentration von 21% (Nomoxia Gasgemisch) nur zu einer geringen Bildung von Reaktiven Sauerstoffmolekülen. Unter Sauer-' stoffmangel (0,1% Sauerstoff, Hypoxia Gasgemisch) kommt es zu einer 3-fach höheren Bildung von Reaktiven Sauerstoffmolekülen. Wird jedoch das Peptid SEQ ID No. 1 zu Endothelzellen, welche unter Sauerstoffarmut (Sauerstoffgehalt 0,1%, Hypoxia Gasgemisch) kultiviert werden, hinzugegeben, so werden keine Reaktiven Sauerstoffmoleküle durch die Endothelzellen gebildet.

Im Gegensatz zu Peptid SEQ ID No. 1 führt eine Zugabe von Peptid SEQ ID No. 3 zu Endothelzellen, welche unter Sauerstoffarmut (Sauerstoffgehalt 0,1%, Hypoxia Gasgemisch) kultiviert werden, zu keiner Hemmung der Bildung von Reaktiven Sauerstoffmolekülen durch die Endothelzellen.

Der Unterschied zwischen Peptid SEQ ID No. 3 zum Peptid SEQ ID No. 1 besteht darin, dass die Aminosäuren Thr(6), Glu(8), Glu(11) aus Peptid SEQ ID No. l in Ala(6), Ala(8) und Ala(11) im Peptid SEQ ID No. 3 vorliegen.

### Beispiel 3: Hemmung der Hyperpermeabilität in Endothelzellen und Epithelzellen durch das Peptid SEQ ID No. 1

### Materialien

Menschliche Epithelzellen der Lunge Typ H441 wurden von der Firma ATTC bezogen.

Menschliche Endothelzellen der Lunge, isoliert aus Kapillaren der Lunge, wurden von der Firma Lonza bezogen.

Die mikrobiellen Toxine Listeriolysin (LLO) und Pneumolysin (PLY) wurden von der Universität Giessen bezogen.

### Zellkultur

Menschliche Endothelzellen der Lunge, isoliert aus Kapillaren der Lunge, wurden in laborüblicher Weise kultiviert.

Epithelzellen der Lunge Typ H441 wurden in laborüblicher Weise in einem kommerziellen RPMI 1640 Medium mit den Zusätzen 2 mM L-Glutamin, 1,5 g/l Natriumbikarbonat, 4,5 g/l Glukose, 10 mM HEPES Puffer pH 7,4, 10% Kälberserum, kultiviert. Die ECIS Experimente erfolgten in Serum-freiem Medium.

### Hyperpermeabilität

Zur Herbeiführung einer Hyperpermeabilität, d.h. Verletzungen der Endothelzellen und Epithelzellen, wurden die menschlichen Epithelzellen der Lunge, sowie die menschlichen Endothelzellen der Lunge in laborüblicher Weise bis zur Bildung eines durchgängigen Zellrasens kultiviert, und anschließend wurden die Toxine Listeriolysin bzw. Pneumolysin zugegeben.

### Bestimmung des transendothelialen Widerstandes

Vor, während und nach der Zugabe der mikrobiellen Toxine Pneumolysin und Listeriolysin zu menschlichen Endothelzellen wurde der elektrische Widerstand der Zellschicht (trans-endothelialer Widerstand) mittels elektrischer Zell-Substrat Impedanzanalyse bestimmt.

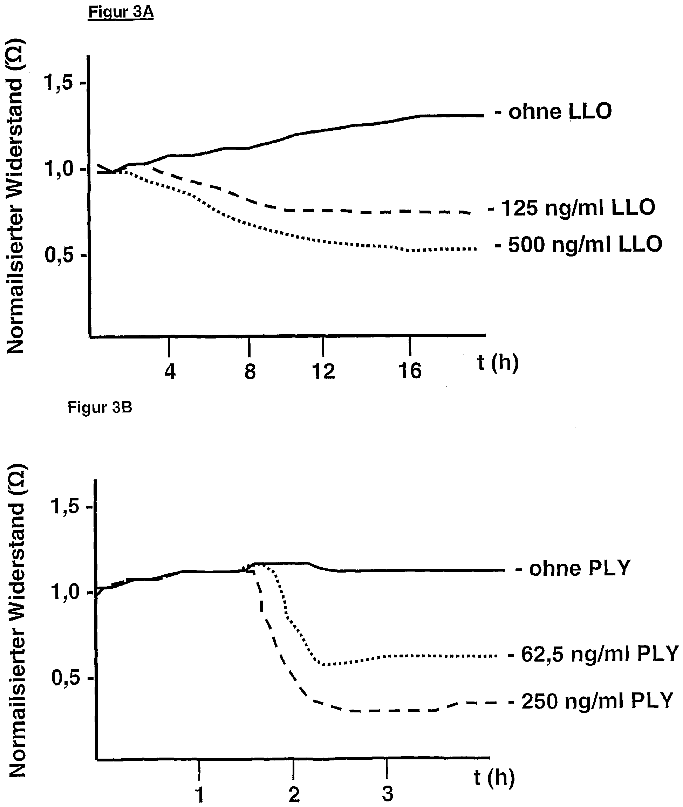

Wie die Figur 3A zeigt, nimmt mit einer Zugabe von 125 ng/ml Listeriolysin zu kultivierten menschlichen Endothelzellen der elektrische Widerstand ab. Es kommt zur Herausbildung einer Hyperpermeabilität. Dieser Effekt ist bei einer höheren Menge von 500 ng/ml Listeriolysin noch deutlicher.

Wie die Figur 3B zeigt, nimmt mit einer Zugabe von 62,5 ng/ml Pneumolysin zu kultivierten menschlichen Endothelzellen der elektrische Widerstand ab. Es kommt zur Herausbildung einer Hyperpermeabilität. Dieser Effekt ist bei einer höheren Menge von 250 ng/ml Pneumolysin noch deutlicher.

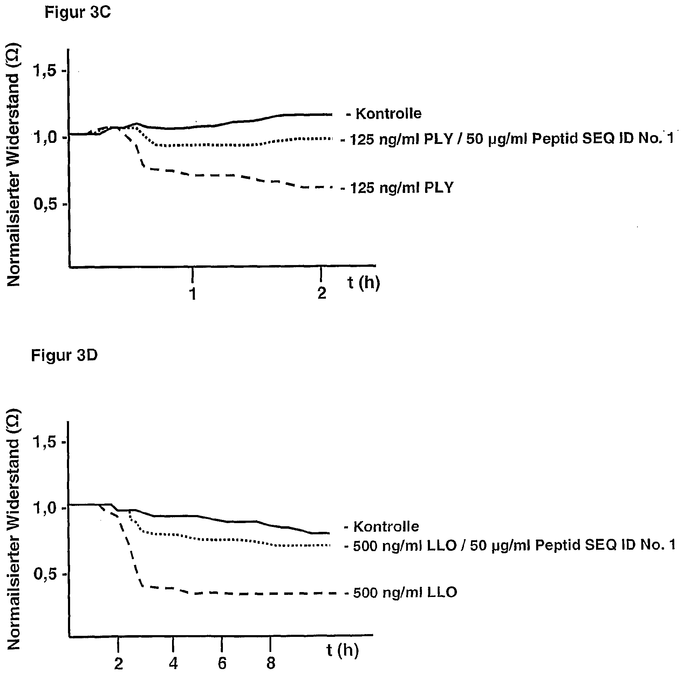

Wie die Figur 3C zeigt, nimmt mit einer Zugabe von 125 ng/ml Pneumolysin zu kultivierten menschlichen Endothelzellen der elektrische Widerstand ab. Es kommt zur Herausbildung einer Hyperpermeabilität. Die durch Zugabe des Toxin Pneumolysin hervorgerufene Hyperpermeabilität wird jedoch durch eine Zugabe von 50 ug/ml Peptid SEQ ID No. 1 inhibiert.

Wie die Figur 3D zeigt, nimmt mit einer Zugabe von 500 ng/ml Listeriolysin zu kultivierten menschlichen Endothelzellen der elektrische Widerstand ab. Es kommt zur Herausbildung einer Hyperpermeabilität. Die durch Zugabe des Toxins Listeriolysin hervorgerufene Hyperpermeabilität wird jedoch durch eine Zugabe von 50 µg/ml Peptid SEQ ID No. 1 inhibiert.

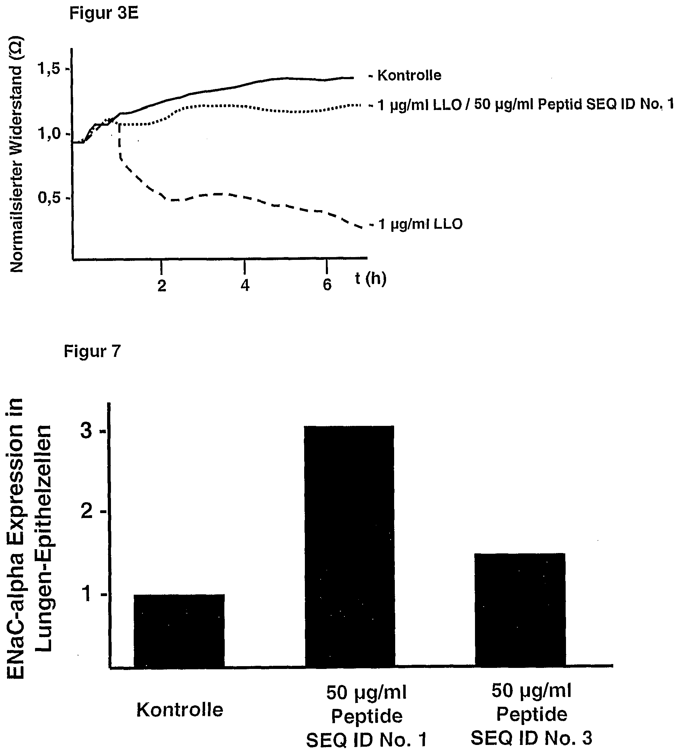

Wie die Figur 3E zeigt, nimmt mit einer Zugabe von 1 µg/ml Listeriolysin zu kultivierten menschlichen Epithelzellen der elektrische Widerstand ab. Es kommt zur Herausbildung einer Hyperpermeabilität. Die durch Zugabe des Toxins Listeriolysin hervorgerufene Hyperpermeabilität wird jedoch durch eine Zugabe von 50 ug/ml Peptid SEQ ID No. 1 inhibiert.

### Beispiel 4: Hemmung der Phosphorylierung der Myosin Light Chain durch das Peptid SEQ ID No. 1

### Materialien

Menschliche Endothelzellen der Lunge, isoliert aus Kapillaren der Lunge, wurden von der Firma Lonza bezogen.

Die mikrobiellen Toxine of Listeriolysin (LLO) und Pneumolysin (PLY) wurden von der Universität Giessen bezogen.

### Zellkultur

Menschliche Endothelzellen der Lunge, isoliert aus Kapillaren der Lunge, wurden in laborüblicher Weise kultiviert.

### Bestimmung der Phosphorylierung der Myosin Light Chain

Zur Bestimmung der Phosphorylierung der Myosin Light Chain, und dem Einfluss des Peptides SEQ ID No. 1 auf die Phosphorylierung, wurden die zuvor kultivierten menschlichen Endothelzellen der Lungen mit Phosphatpuffer pH 7,4 welcher 1 mM Orthovanadate enthielt gewaschen. Der Zellinhalt wurde durch Inkubation der Zellen mit einer Lösung aus 20 mM Tris Puffer (pH 7,4), 150 mM mol/l NaCl, 1 mM EDTA, l mM EGTA, 1% Triton X-100, 2,5 mM Natrium Pyrophosphat, 1 mM beta-Glycerophosphat, 1 mM Natriumvanadat, 1 µg/ml Leupeptin, 1 mM Phenylmethylsulfonylfluorid lysiert. Zusätzlich wurden die Zellen durch Ultraschall aufgeschlossen. Das Zelllysat wurde zentrifugiert um die löslichen Bestandteile zu gewinnen. Das lösliche Zelllysat wurde anschließend in laborüblicher Weise auf eine denaturierende Natriumdocecylsulfate Polyacrylamidgel Elektrophorese aufgetragen und die Proteine wurden entsprechend ihrer Masse aufgetrennt. Danach wurden die Proteine auf Nitrozellulosemembranen übertragen. Die Protein Blots wurden in laborüblicher Weise mit einer Lösung aus 0,1% Tween 20 und 5% Trockenmilchpulver für 1 Stunde behandelt. Anschließend wurden die Protein Blots mit Antikörpern, welche entweder gegen die Myosin Light Chain gerichtet sind, oder welche gegen phosphorylierte Myosin Light Chain gerichtet sind inkubiert.

Zur Sichtbarmachung der entweder Myosin Light Chain oder der phosphorylierten Myosin Light Chain wurden die Antikörper in laborüblicher Weise mittels Chemolumineszenz auf diagnostischem Film sichtbar gemacht. Die Signalstärke wurde durch Densitometrie ermittelt und das Verhältnis aus Myosin Light Chain zu phosphorylierter Myosin Light Chain wurde ermittelt.

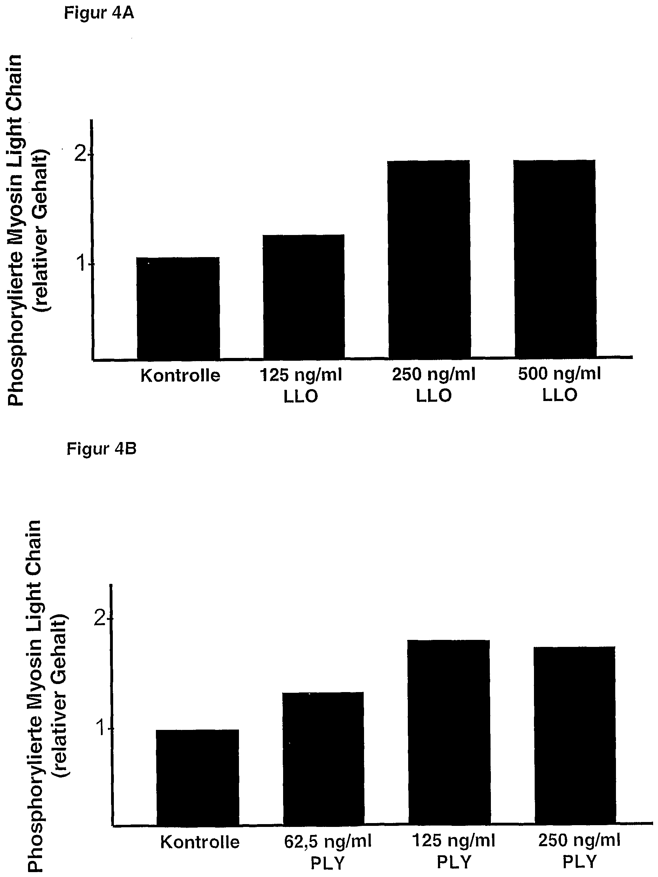

Wie die Figur 4A zeigt, führt eine Zugabe des Toxins 125 ng/ ml Listeriolysin zu menschlichen endothelialen Lungenzellen zu einer Zunahme des relativen Gehalts an phosphorylierter Myosin Light Chain. Dieser Effekt wird durch eine Toxinkonzentrati-on von 250 ng/ml Listeriolysin noch verstärkt.

Wie die Figur 4B zeigt, führt eine Zugabe des Toxins 62,5 ng/ml Pneumolysin zu menschlichen endothelialen Lungenzellen zu einer Zunahme des relativen Gehalts an phosphorylierter Myosin Light Chain. Dieser Effekt wird durch eine Toxinkonzentration von 125 ng/ml Pneumolysin noch verstärkt.

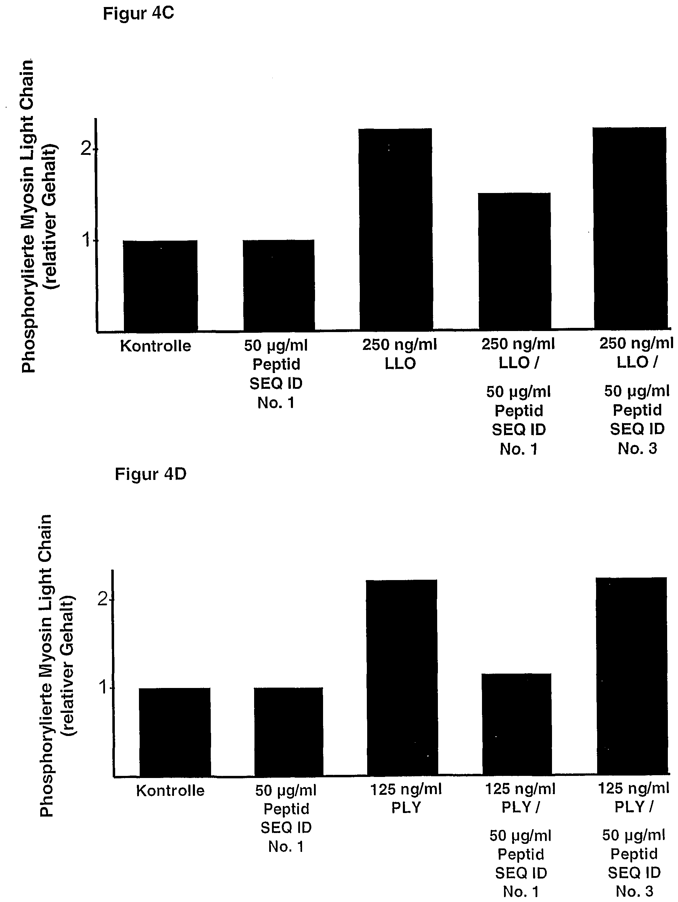

Wie die Figur 4C zeigt, führt eine Zugabe des Toxins 125 ng/ ml Listeriolysin zu menschlichen endothelialen Lungenzellen zu einer Zunahme des relativen Gehalts an phosphorylierter Myosin Light Chain. Eine Zugabe von 50 µg/ml des Peptides SEQ ID No. 1 hat keinen Einfluss auf den Gehalt von phosphorylierter Myosin Light Chain. Die Erhöhung des Gehalts an phosphorylierter Myosin Light Chain durch 250 ng/ml Toxin Listeriolysin wird durch eine Zugabe von 50 µg Peptid SEQ ID No. 1 inhibiert. Ein Peptid SEQ ID No. 3 hat keinen Einfluss auf die durch das Toxin Listeriolysin vermittelte Erhöhung des Gehaltes an phosphorylierter Myosin Light Chain.

Wie die Figur 4D zeigt, führt eine Zugabe des Toxins 125 ng/ ml Pneumolysin zu menschlichen endothelialen Lungenzellen zu einer Zunahme des relativen Gehalts an phosphorylierter Myosin Light Chain. Eine Zugabe von 50 µg/ml des Peptides SEQ ID No. 1 hat keinen Einfluss auf den Gehalt von phosphorylierter Myosin Light Chain. Die Erhöhung des Gehalts an phosphorylierter Myosin Light Chain durch 125 ng/ml Toxin Pneumolysin wird durch eine Zugabe von 50 µg Peptid SEQ ID No. 1 inhibiert. Ein Peptid SEQ ID No. 3 hat keinen Einfluss auf die durch das Toxin Pneumolysin-vermittelte Erhöhung des Gehalts an phosphorylierter Myosin Light Chain.

Der Unterschied zwischen Peptid SEQ ID No. 3 zum Peptid SEQ ID No. 1 besteht darin, dass die Aminosäuren Thr(6), Glu(8), Glu(11) aus Peptid SEQ ID No. 1 in Ala(6), Ala(8) und Ala(11) im Peptid SEQ ID No. 3 vorliegen.

### Beispiel 5: Einfluss des Peptides SEQ ID No. 1 auf die Hyperpermeabilität und den akuten Lungenschaden im Tiermodel

### Induktion der Hyperpermeabilität in Mäusen

Labormäuse wurden mit einer Mischung aus Isoflura-ne/Sauerstoff vor der Präparation der Lungen, sowie mit 100 µl pro Maus einer Mischung aus Ketamin/Rompun (1,33:1), intratracheal behandelt. Nach der Narkose wurde den Mäusen ein venöser Katheter implantiert. Zur Induktion einer Hyperpermeabilität der Lunge wurden mit einer feinen Spritze anschließend 25 µl Flüssigkeit. in die Lunge vernebelt. Die Flüssigkeit enthielt entweder 0,9% Kochsalzlösung, oder 250 ng Toxin Pneumolysin, oder 250 ng/ml Pneumolysin/50 µg/ml Peptid SEQ ID No. 1.

### Visualisierung der Hyperpermeabilität durch Evans Blau

5,5 Stunden nach der Verabreichung des Toxins Pneumolysin wurde den Mäusen der Farbstoff Evans Blau, gelöst in 0,9% Kochsalzlösung, zu 100 mg/kg Mausgewicht intravenös appliziert. Nach 30 Minuten wurde den Tieren durch Herzpunktion das Blut entnommen. Anschließend wurde die Lunge entnommen, mit 1 ml EDTA-Phosphatpuffer (pH 7,4) gewaschen und in flüssigem Stickstoff schockgefroren. Zur Bestimmung des Gehalts an Evans Blau Farbstoff im Lungengewebe wurden die Lungen dann in kaltem Phosphatpuffer homogenisiert (1 ml Puffer pro 100 mg Lunge), mit Formalinlösung für 18 Stunden inkubiert und anschließend zentrifugiert (5.000 x g, 30 Minuten). Im Flüssigkeitsüberstand wurde dann die Absorptionen bei 620 nm und bei 740 nm photometrisch bestimmt. Der Gehalt an Evans Blau Farbstoff im Lungengewebe wurde auf der Basis einer Referenzkurve für Evans Blau Farbstoff gelöst in Formalinlösung, und unter Abzug des Gehalts an Hämoglobin-Pigmenten ermittelt. Der Austritt von Evans Blau Fabstoff aus den Kapillaren in das Lungengewebe durch die mittels des Toxin Pneumolysin induzierte Hyperpermeabilität wurde in Relation zur Farbstoffmenge im Blutserum gesetzt.

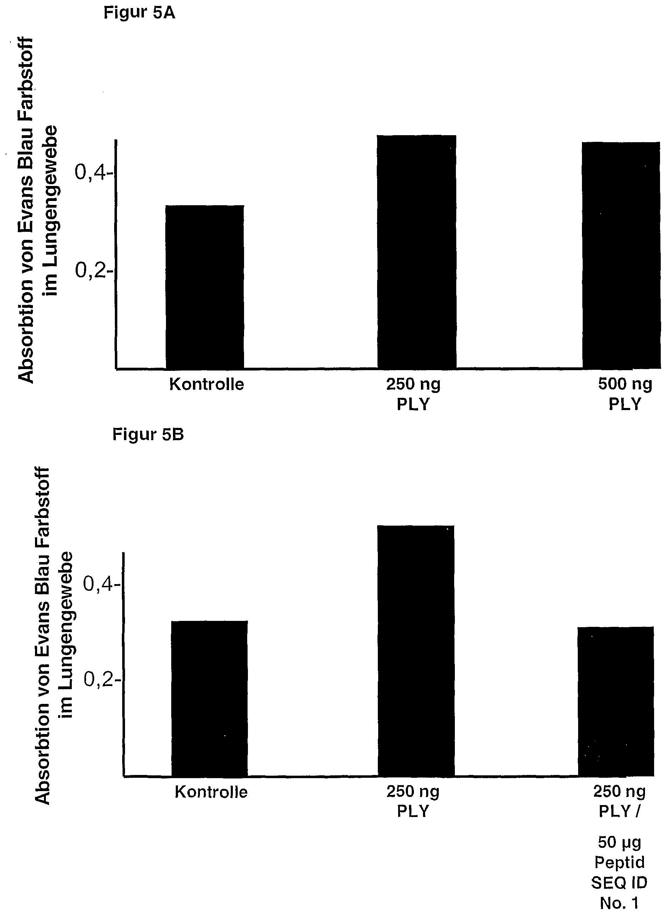

Wie die Figur 5A zeigt, führt eine intratracheale Applikation des Toxins Pneumolysin mit einer Dosis von 250 ng und 500 ng pro Maus zu einer Hyperpermeabilität, bestimmt dadurch, dass der Farbstoff Evans Blau Blut aus den Lungenkapillaren in das Lungengewebe übertritt, und in dem Lungengewebe nachgewiesen werden kann.

Wie die Figur 5B zeigt, führt eine intratracheale Applikation des Toxins Pneumolysin mit einer Dosis von 250 ng pro Maus zu einer Hyperpermeabilität, bestimmt dadurch, dass der Farbstoff Evans Blut aus den Lungenkapillaren in das Lungengewebe übertritt, und in dem Lungengewebe nachgewiesen werden kann. Durch die intratracheale Applikation von 50 µg Peptid SEQ ID No. 1 kommt es zur Hemmung der Toxin-bedingten Herausbildung der Hyperpermeabilität .

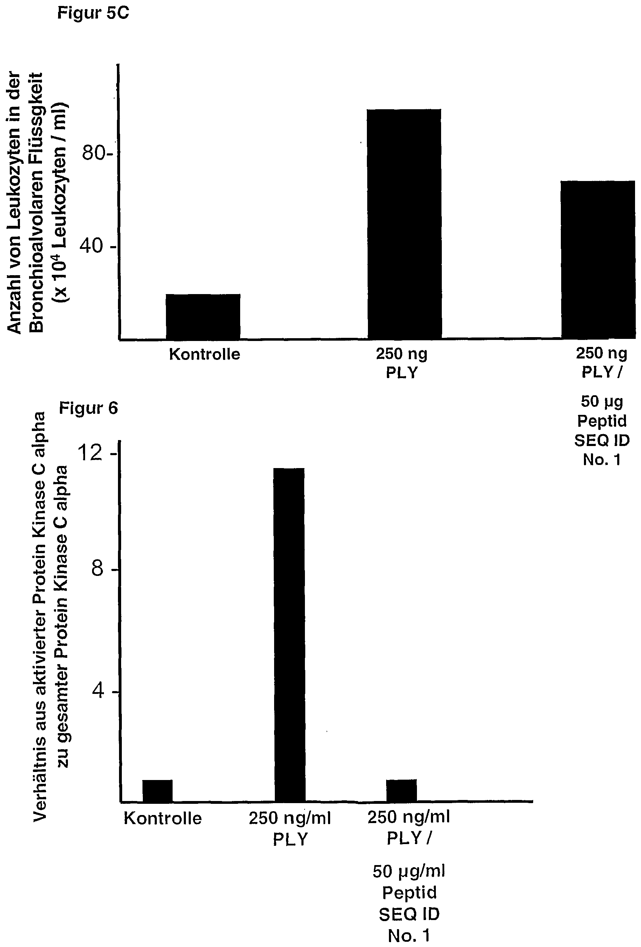

Wie die Figur 5C zeigt, führt eine intratracheale Applikation des Toxins Pneumolysin mit einer Dosis von 250 ng pro Maus durch die Ausprägung einer Hyperpermeabilität zu einer erhöhten Anzahl von Leukozyten in der bronioalvolaren Flüssigkeit von Lungen in Mäusen. Durch die intratracheale Applikation von 50 µg Peptid SEQ ID No. 1 kommt es zur Hemmung der Toxin-bedingten Herausbildung der Hyperpermeabilität und einer deutlichen Verringerung der Anzahl der Leukozyten in bronioalvolarer Flüssigkeit in Lungen von Mäusen.

### Beispiel 6: Hemmung der Aktivierung der Protein Kinase C durch Peptid der SEQ ID No. 1

### Materialien

Menschliche Endothelzellen der Lunge, isoliert aus Kapillaren der Lunge, wurden von der Firma Lonza bezogen.

Das mikrobielle Toxin Pneumolysin (PLY) wurde von der Universität Giessen bezogen.

### Zellkultur

Menschliche Endothelzellen der Lunge, isoliert aus Kapillaren der Lunge, wurden in laborüblicher Weise kultiviert. Während der Zellkultur wurde das Toxin Pneumolysin mit einer Konzentration von 250 ng/ml, oder das Toxin Pneumolysin mit einer Konzentration von 250 ng/ml und das Peptid SEQ ID No. 1 mit einer Konzentration von 50 µg/ml zugegeben.

Bestimmung des Gehaltes an aktivierter Protein Kinase C alpha Der Gehalt der aktivierten Protein Kinase C alpha wurde durch ELISA-Messung unter Verwendung eines gegen die aktivierte Protein Kinase C alpha (phospho-Threonin 638 Protein Kinase C alpha) gerichteten Antikörpers bestimmt. Gleichzeitig wurde der Gesamtgehalt an Protein Kinase C alpha mittels handelsüblichem ELISA-Assay bestimmt.

Wie die Figur 6 zeigt, kommt es durch die Wirkung des Toxins Pneumolysin zu einem starken Anstieg des Gehalts an aktivierter Protein Kinase C alpha im Vergleich zur Gesamtkonzentration an Protein Kinase C alpha. Durch die Zugabe von Peptid SEQ ID No. 1 kommt es zu einer Hemmung der Aktivierung der Protein Kinase C alpha.

### Beispiel 7: Erhöhung der Expression des epithelialen Natriumkanals (ENaC) in Epithelzellen durch das Peptid SEQ ID No. 1

### Materialien

Menschliche Epithelzellen der Lunge Typ H441 wurden von der Firma ATTC bezogen.

### Zellkultur

Epithelzellen der Lunge Typ H441 wurden in laborüblicher Weise in einem kommerziellen RPMI 1640 Medium mit den Zusätzen 2 mM L-Glutamin, 1,5 g/l Natriumbikarbonat, 4,5 g/l Glukose, 10 mM HEPES Puffer pH 7,4, 10% Kälberserum, kultiviert.

### Nachweis der Expression des epithelialen Natriumkanals

In den kultivierten Epithelzellen wurde die Expression des Natriumkanals (ENaC) mittels "Real-Time PCR" bestimmt. Diese Untersuchungen erfolgten an Zellen ohne und mit Zugabe von 50 ug/ml Peptid SEQ ID No. 1, sowie nach Zugabe von 50 µg/ml Peptid SEQ ID No. 3.

Wie die Untersuchung 7 zeigt, kommt es durch Zugabe von 50 ug/ml Peptid SEQ ID No. 1 zu Epithelzellen der Lunge zu einer Verdreifachung der Expression des Natriumkanals ENaC.

Durch eine Zugabe von 50 µg/ml Peptid SEQ ID No. 3 kommt es zu keiner wesentlichen Erhöhung der Expression des Natriumkanals ENaC.

Der Unterschied zwischen Peptid SEQ ID No. 3 zum Peptid SEQ ID No. 1 besteht darin, dass die Aminosäuren Thr(6), Glu(8), Glu(11) aus Peptid SEQ ID No. 1 in Ala(6), Ala(8) und Ala(11) im Peptid SEQ ID No. 3 vorliegen.

### Beispiel 8: Wirkung des Peptides SEQ ID No. 1 auf den Krankheitsverlauf von Mäusen mit viraler Lungeninfektion

Es wurden folgende Tier-Studiengruppen hinsichtlich der Wirkung des Peptides SEQ ID No. 1 auf eine virale Lungeninfektion untersucht:
Gruppe 1. Negative Kontrolle (PBS per nasal).
Gruppe 2. Positive Kontrolle (Infektion mit ca. 2.000 Einheiten Influenza A Virus per nasal).
Gruppe 3. Test Gruppe (Infektion mit ca. 2.000 Einheiten Influenza A Virus per nasal, sowie intratracheale Verabreichung von 10 µg Peptid SEQ ID No. 1).

Je Gruppe wurden 6 BALB/c Mäuse verwendet.
Es wurde nach folgendem Behandlungsschema vorgegangen:
Behandlungstag 0:
   Gruppe 1: Verabreichung von PBS per nasal.
   Gruppe 2: Infektion der Mäuse mit Influenza Virus A per nasal.
   Gruppe 3: Infektion der Mäuse mit Influenza Virus A per nasal und Peptid SEQ ID No. 1.
Behandlungstage 0, 2, 4, 6, 8:
   Gruppe 1: Intratracheale Verabreichung von PBS.
   Gruppe 2: Intratracheale Verabreichung von PBS.
   Gruppe 3: Intratracheale Verabreichung von Peptid SEQ ID No. 1.
Behandlungstage 0 bis 10:
   Tägliche Beobachtung von Körpertemperatur, Körpergewicht und Überlebensrate der Testtiere.

Die Untersuchungen ergaben, dass Testtiere mit viraler Lungeninfektion (Gruppe 2) innerhalb von 10 Tagen ca. 20% ihres Körpergewichtes verloren.

Demgegenüber verringerte sich das Körpergewicht der Testtiere nur um ca. 10%, wenn das Peptid SEQ ID No. 1 verabreicht wurde (Gruppe 3).

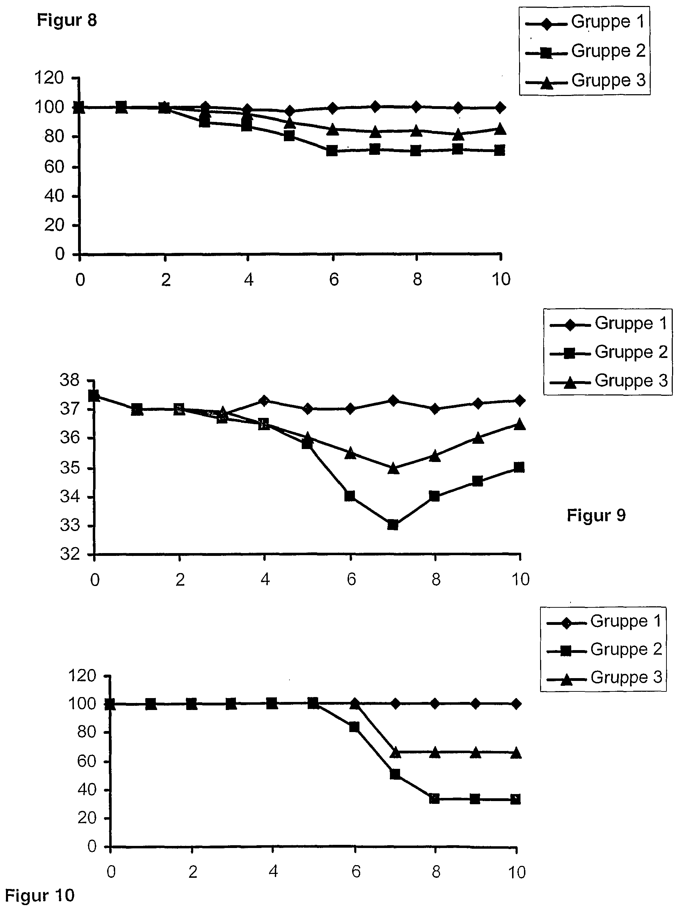

Die Ergebnisse sind in Figur 8 dargestellt.

Die Untersuchungen ergaben außerdem, dass sich bei Testtieren mit viraler Lungeninfektion (Gruppe 2) nach 7 Tagen die Körpertemperatur von 37,5°C auf 33°C abkühlte. Anschließend stieg die Körpertemperatur auf 35°C.

Demgegenüber verringerte sich bei Testtieren mit Verabreichung des Peptides SEQ ID No. 1 (Gruppe 3) nach 7 Tagen nur auf 35°C. Anschließend stieg die Körpertemperatur wieder auf 37°C.

Die Ergebnisse sind in Figur 9 dargestellt.

Die Untersuchungen ergaben außerdem, dass 10 Tage nach viraler Lungeninfektion 2/3 der Testtiere der Gruppe 2 gestorben waren.

Demgegenüber war die Sterblichkeit von Testtieren bei Verabreichung von Peptid SEQ ID No. 1 (Gruppe 3) nach 10 Tagen nur 1/3.

Die Ergebnisse sind in Figur 10 dargestellt.

Insgesamt zeigen die Untersuchungen an Testtieren mit viraler Lungeninfektion, dass die Verabreichung des Peptides SEQ ID No. 1 sowohl die Abnahme des Körpergewichtes verringert, das Absinken der Körpertemperatur mindert und zu einer deutlich erhöhten Überlebensrate führt.

### Beispiel 9: Anwendung des Peptides SEQ ID No. 1 ("AP301") in einem Lavage-induzierten ARDS-Großtiermodell

Material & Methoden: Mit Genehmigung der zuständigen Tierschutzkommission wurde bei zwei Schweinen (25kg) in Allgemeinanästhesie ein Lungenschaden durch Surfactantdepletion (viermalige bronchoalveolärer Lavage, je 30ml/kg KG) induziert. Anschließend wurde 1 mg/kg KG AP301 (Peptid SEQ ID No.1) endotracheal appliziert. Tier 1 (1) erhielt eine tief tracheale Injektion der Gesamtdosis, bei Tier 2 (2) wurde eine Verneblung derselben Dosierung über 30 min durchgeführt. Daraufhin erfolgte eine fünfstündige Beatmungszeit. Der arterielle Sauerstoffpartialdruck (paO₂) wurde mittels einer zuvor validierten intraaortalen Echtzeit-Messsonde (FOXY, Ocean Optics, USA) erfasst. Spirometrie und Hämodynamik wurden permanent registriert sowie in halbstündigen Intervallen Messungen mit der PiCCO-Technologie durchgeführt.

Ergebnisse: Es zeigten sich keine unerwünschten hämodynamischen Wirkungen während der Applikation des Medikamentes. Beatmungseinstellungen wurden konstant im nicht-protektiven Bereich gehalten (Pmax 40 mbar, Tidalvolumen ≥ 10ml/kg KG, PEEP ≤ 10 mbar, Frequenz 25-35/min) um therapeutische Effekte zu vermeiden. Bei beiden Tieren zeigte sich eine kontinuierlich auf etwa 1,5 Stunden limitierte Verbesserung der Oxygenierung mit einem paO₂ Anstieg um max. 162,8 mmHg (1) bzw. 224,6 mmHg (2). Diese trat bei Verneblung von AP301 zeitlich verzögert im Vergleich zur tief trachealen Applikation auf, war jedoch ausgeprägter. Parallel zur Verbesserung des Gasaustausches konnte eine Reduktion des extravaskulären Lungenwassers um 15,8-52,5% zum Ausgangswert nach Surfactantdepletion registriert werden.

Diese Ergebnisse zeigen eindrucksvoll, dass sich der erfindungsgemäße neue pharmakologische Wirkansatz bei der Behandlung des ARDS auch im anerkannten Großtiermodell zur Behandlung von ARDS als effizient beweist.

### Zusammenfassung der Sequenzen:

SEQ ID No. 1 CGQRETPEGAEAKPWYC
SEQ ID No. 2 KSPGGQRETPEGAEAKPWYE
SEQ ID No. 3 CGQREAPAGAAAKPWYC
SEQ ID No. 4 TPEGAE
SEQ ID No. 5 QRETPEGAEAKPWY
SEQ ID No. 6 PKDTPEGAELKPWY
SEQ ID No. 7 CGPKDTPEGAELKPWYC
SEQ ID No. 8 CGQKETPEGAEAKPWYC
SEQ ID No. 9 CGQRETPEGAEARPWYC
SEQ ID No. 10 CGQRETPEGAEAKPC
SEQ ID No. 11 CQRETPEGAEAKPWYC
SEQ ID No. 12 CGQRETPEGAEAKFWYC

## Patentansprüche

1. Peptid, welches aus 7-17 benachbarten Aminosäuren besteht und das Hexamer TX₁EX₂X₃E umfasst, wobei X₁, X₂ und X₃ jede natürliche oder nicht natürliche Aminosäure sein kann, wobei das Peptid keine TNF-Rezeptor-Bindungsaktivität aufweist und zyklisiert ist, zur Anwendung zur Verhinderung von Ödemen durch Verringerung der Hyperpermeabilität, beruhend auf Verletzungen der Endothel- und Epithelschichten.

2. Peptid, welches aus 7-17 benachbarten Aminosäuren besteht und das Hexamer TPEGAE umfasst, wobei das Peptid keine TNF-Rezeptor- Bindungsaktivität aufweist und zyklisiert ist, zur Anwendung zur Verhinderung von Ödemen durch Verringerung der Hyperpermeabilität, beruhend auf Verletzungen der Endothel- und Epithelschichten .

3. Zyklisiertes Peptid, bestehend aus einer Sequenz aufeinander folgender Aminosäuren, ausgewählt aus der Gruppe bestehend aus
- QRETPEGAEAKPWY
- PKDTPEGAELKPWY
- CGQRETPEGAEAKPWYC und
- CGPKDTPEGAELKPWYC
und Fragmenten von mindestens 7 Aminosäuren davon, welche Fragmente das Hexamer TPEGAE aufweisen, zur Herstellung eines Medikaments zur Anwendung zur Verhinderung von Ödemen durch Verringerung der Hyperpermeabilität, beruhend auf Verletzungen der Endothel- und Epithelschichten .

4. Peptid, wie in einem der Ansprüche 1 bis 3 definiert, zur Anwendung zur Verhinderung von Ödemen bei Behandlung von Pneumonie, akuter Lungenverletzung, Akutem Respiratorischen Distress-Syndrom (ARDS) oder bakteriellen oder viralen Lungenkrankheiten, insbesondere Infektionen mit Listeria monocytogenes, Streptococcus pneumoniae, SARS-Viren, RSV oder Influenza-Viren.

5. Peptid zur Anwendung nach einem der Ansprüche 1 bis 4, **dadurch gekennzeichnet, dass** es die Aminosäuresequenz CGQRETPEGAEAKPWYC umfasst und über die C-Reste zyklisiert ist.

6. Peptid zur Anwendung nach Anspruch 5, **dadurch gekennzeichnet, dass** es durch eine Disulfidbrücke zwischen den C-Resten zyklisiert ist.

7. Peptid zur Anwendung nach einem der Ansprüche 1 bis 6, **dadurch gekennzeichnet, dass** es zum Schutz von Epithelzellen und Endothelzellen vor Hyperpermeabilität, ausgelöst durch Reaktive Sauerstoffmoleküle, verwendet wird.

8. Peptid zur Anwendung nach einem der Ansprüche 1 bis 6, **dadurch gekennzeichnet, dass** es zum Schutz von Epithelzellen und Endothelzellen vor Hyperpermeabilität, ausgelöst durch bakterielle Toxine, verwendet wird.

9. Peptid zur Anwendung nach einem der Ansprüche 1 bis 6, **dadurch gekennzeichnet, dass** es zur Hemmung der Phosphorylierung der Myosin Light Chain verwendet wird.

10. Peptid zur Anwendung nach einem der Ansprüche 1 bis 6, **dadurch gekennzeichnet, dass** es zur Hemmung der Aktivierung der Protein Kinase C verwendet wird.

11. Peptid zur Anwendung nach einem der Ansprüche 1 bis 6, **dadurch gekennzeichnet, dass** es zur Erhöhung der Expression des epithelialen Natriumkanals verwendet wird.

12. Peptid nach einem der Ansprüche 1 bis 6, **dadurch gekennzeichnet, dass** es zur Verhinderung von Ödemen durch Verringerung einer Hyperpermeabilität, ausgelöst durch Reaktive Sauerstoffmoleküle, mikrobielle Toxine,
Gram-positive Mikroorganismen oder durch pulmonalen Virenbefall, verwendet wird.

13. Pharmazeutische Zusammensetzung zur Anwendung zur Verhinderung von Ödemen durch Verringerung der Hyperpermeabilität, beruhend auf Verletzungen der Endothel- und Epithelschichten, enthaltend ein Peptid nach einem der Ansprüche 1 bis 12 und einen pharmazeutischen Träger.

## Claims

1. A peptide, which consists of 7-17 adjacent amino acids and includes the hexamer TX₁EX₂X₃E, wherein X₁, X₂ and X₃ can be any natural or non-natural amino acid, wherein the peptide has no TNF-receptor binding activity and is cyclicized, for use in preventing oedemas by reducing the hyperpermeability, due to damage to the endothelial and epithelial layers.

2. A peptide, which consists of 7-17 adjacent amino acids and includes the hexamer TPEGAE, wherein the peptide has no TNF-receptor binding activity and is cyclicized, for use in preventing oedemas by reducing the hyperpermeability, due to damage to the endothelial and epithelial layers.

3. A cyclicized peptide, consisting of a sequence of successive amino acids selected from the group consisting of
- QRETPEGAEAKPWY
- PKDTPEGAELKPWY
- CGQRETPEGAEAKPWYC and
- CGPKDTPEGAELKPWYC
and fragments of at least 7 amino acids thereof, which fragments include the hexamer TPEGAE, for producing a medicament for use in preventing oedemas by reducing the hyperpermeability, due to damage to the endothelial and epithelial layers.

4. A peptide as defined in one of Claims 1 to 3, for use in preventing oedemas in the treatment of pneumonia, acute lung damage, acute respiratory distress syndrome (ARDS) or bacterial or viral lung diseases, particularly infections with Listeria monocytogenes, Streptococcus pneumoniae, SARS viruses, RSV or influenza viruses.

5. A peptide for use as claimed in one of Claims 1 to 4, **characterised in that** it includes the amino acid sequene CGQRETPEGAEAKPWYZ and is cyclized via the C residues.

6. A peptide for use as claimed in Claim 5, **characterised in that** it is cyclicized by a disulphide bridge between the C residues.

7. A peptide for use as claimed in one of Claims 1 to 6, **characterised in that** it is used for protecting epithelial cells and endothelial cells from hyperpermeability, initiated by reactive oxygen molecules.

8. A peptide for use as claimed in one of Claims 1 to 6, **characterised in that** it is used for protecting epithelial cells and endothelial cells from hyperpermeability, initiated by bacterial toxins.

9. A peptide for use as claimed in one of Claims 1 to 6, **characterised in that** it is used for inhibiting phosphorylation of the Myosin Light Chain.

10. A peptide for use as claimed in one of Claims 1 to 6, **characterised in that** it is used for inhibiting the activation of the protein kinase C.

11. A peptide for use as claimed in one of Claims 1 to 6, **characterised in that** it is used for increasing the expression of the epithelial sodium channel.

12. A peptide as claimed in one of Claims 1 to 6, **characterised in that** it is used for preventing oedemas by reducing a hyperpermeability, initiated by reactive oxygen molecules, microbial toxins, gram-positive microorganisms or by pulmonary virus attack.

13. A pharmaceutical composition for use for preventing oedemas by reducing the hyperpermeability, based on damage to the endothelial and epithelial layers, containing a peptide as claimed in one of Claims 1 to 12 and a pharmaceutical carrier.

## Revendications

1. Peptide qui est constitué de 7 à 17 acides aminés voisins et qui comprend l'hexamère TX₁EX₂X₃E, où X₁, X₂ et X₃ peuvent être chacun chaque acide aminé naturel ou non naturel, le peptide ne présentant pas d'activité de liaison des récepteurs TNF et étant cyclisé, pour une utilisation dans le but de prévenir des oedèmes par diminution de l'hyperperméabilité, se fondant sur des lésions des couches endothéliales et épithéliales.

2. Peptide qui est constitué de 7 à 17 acides aminés voisins et comprend l'hexamère TPEGAE, le peptide ne présentant pas d'activité de liaison des récepteurs TNF et étant cyclisé, pour une utilisation dans le but de prévenir des oedèmes par diminution de l'hyperperméabilité, se fondant sur des lésions des couches endothéliales et épithéliales.

3. Peptide cyclisé, constitué d'une séquence d'acides aminés successifs, choisi dans le groupe consistant en
- QRETPEGAEAKPWY
- PKDTPEGAELKPWY
- CGQRETPEGAEAKPWYC et
- CGPKDTPEGAELKPWYC
et de fragments d'au moins 7 acides aminés de ceux-ci, lesquels fragments présentant l'hexamère TPEGAE, pour la fabrication d'un médicament destiné à une utilisation dans le but de prévenir des oedèmes par diminution de l'hyperperméabilité, se fondant sur des lésions des couches endothéliales et épithéliales.

4. Peptide tel que défini dans l'une des revendications 1 à 3, destiné à être utilisé dans le but de prévenir des oedèmes lors du traitement d'une pneumonie, d'une lésion pulmonaire aiguë, du syndrome de détresse respiratoire aiguë (SDRA) ou de maladies pulmonaires bactériennes ou virales, en particulier d'infections à Listeria monocytogenes, à Streptococcus pneumoniae, à virus du SRAS, à VRS ou à virus de la grippe.

5. Peptide destiné à être utilisé selon l'une des revendications 1 à 4, **caractérisé en ce qu'**il comprend la séquence d'acides aminés CGQRETPEGAEAKPWYC et est cyclisé par l'intermédiaire des résidus C.

6. Peptide destiné à être utilisé selon la revendication 5, **caractérisé en ce qu'**il est cyclisé par un pont disulfure entre les résidus C.

7. Peptide destiné à être utilisé selon l'une des revendications 1 à 6, **caractérisé en ce qu'**il est utilisé pour la protection de cellules épithéliales et de cellules endothéliales vis-à-vis d'une hyperperméabilité, déclenchée par des molécules d'oxygène réactives.

8. Peptide destiné à être utilisé selon l'une des revendications 1 à 6, **caractérisé en ce qu'**il est utilisé pour la protection de cellules épithéliales et de cellules endothéliales vis-à-vis d'une hyperperméabilité, déclenchée par des toxines bactériennes.

9. Peptide destiné à être utilisé selon l'une des revendications 1 à 6, **caractérisé en ce qu'**il est utilisé pour inhiber la phosphorylation de la chaîne légère de la myosine.

10. Peptide destiné à être utilisé selon l'une des revendications 1 à 6, **caractérisé en ce qu'**il est utilisé pour inhiber l'activation de la protéine kinase C.

11. Peptide destiné à être utilisé selon l'une des revendications 1 à 6, **caractérisé en ce qu'**il est utilisé pour augmenter l'expression du canal sodique épithélial.

12. Peptide destiné à être utilisé selon l'une des revendications 1 à 6, **caractérisé en ce qu'**il est utilisé pour prévenir des oedèmes par diminution d'une hyperperméabilité, déclenchée par des molécules d'oxygène réactives, des toxines microbiennes, des microorganismes Gram-positives, ou par une infestation virale pulmonaire.

13. Composition pharmaceutique destinée à être utilisée dans le but de prévenir des oedèmes par diminution de l'hyperperméabilité, se fondant sur des lésions des couches endothéliales et épithéliales, contenant un peptide selon l'une des revendications 1 à 12 et un support pharmaceutique.


### SEQUENCE LISTING

<110> Apeptico Forschung und Entwicklung GmbH
<120> verfahren zur vermeidung und zur Behandlung einer
   Hyperpermeabilität
<130> R 55450
<150> A 359/2009
   <151> 2009-03-05
<160> 12
<170> PatentIn version 3.5
<210> 1
   <211> 17
   <212> PRT
   <213> Artificial Sequence
<220>
   <223> synthetic peptide
<400> 1
   Cys Gly Gln Arg Glu Thr Pro Glu Gly Ala Glu Ala Lys Pro Trp Tyr
   1               5                   10                  15
   Cys
<210> 2
   <211> 20
   <212> PRT
   <213> Artificial Sequence
<220>
   <223> synthetic peptide
<400> 2
   Lys Ser Pro Gly Gly Gln Arg Glu Thr Pro Glu Gly Ala Glu Ala Lys
   1               5                   10                  15
   Pro Trp Tyr Glu
               20
<210> 3
   <211> 17
   <212> PRT
   <213> Artificial sequence
<220>
   <223> synthetic peptide
<400> 3
   Cys Gly Gln Arg Glu Ala Pro Ala Gly Ala Ala Ala Lys Pro Trp Tyr
   1 5 10 15
   Cys
<210> 4
   <211> 6
   <212> PRT
   <213> Artificial Sequence
<220>
   <223> synthetic peptide
<400> 4
   Thr Pro Glu Gly Ala Glu
   1 5
<210> 5
   <211> 14
   <212> PRT
   <213> Artificial Sequence
<220>
   <223> synthetic peptide
<400> 5
   Gln Arg Glu Thr Pro Glu Gly Ala Glu Ala Lys Pro Trp Tyr
   1 5 10
<210> 6
   <211> 14
   <212> PRT
   <213> Artificial Sequence
<220>
   <223> synthetic peptide
<400> 6
   Pro Lys Asp Thr Pro Glu Gly Ala Glu Leu Lys Pro Trp Tyr
   1 5 10
<210> 7
   <211> 17
   <212> PRT
   <213> Artificial Sequence
<220>
   <223> synthetic peptide
<400> 7
   Cys Gly Pro Lys Asp Thr Pro Glu Gly Ala Glu Leu Lys Pro Trp Tyr
   1 5 10 15
   Cys
<210> 8
   <211> 17
   <212> PRT
   <213> Artificial Sequence
<220>
   <223> synthetic peptide
<400> 8
   Cys Gly Gln Lys Glu Thr Pro Glu Gly Ala Glu Ala Lys Pro Trp Tyr
   1 5 10 15
   Cys
<210> 9
   <211> 17
   <212> PRT
   <213> Artificial sequence
<220>
   <223> synthetic peptide
<400> 9
   Cys Gly Gln Arg Glu Thr Pro Glu Gly Ala Glu Ala Arg Pro Trp Tyr
   1 5 10 15
   Cys
<210> 10
   <211> 15
   <212> PRT
   <213> Artificial Sequence
<220>
   <223> synthetic peptide
<400> 10
   Cys Gly Gln Arg Glu Thr Pro Glu Gly Ala Glu Ala Lys Pro Cys
   1 5 10 15
<210> 11
   <211> 16
   <212> PRT
   <213> Artificial Sequence
<220>
   <223> synthetic peptide
<400> 11
   Cys Gln Arg Glu Thr Pro Glu Gly Ala Glu Ala Lys Pro Trp Tyr Cys
   1 5 10 15
<210> 12
   <211> 17
   <212> PRT
   <213> Artificial Sequence
<220>
   <223> synthetic peptide
<400> 12
   Cys Gly Gln Arg Glu Thr Pro Glu Gly Ala Glu Ala Lys Phe Trp Tyr
   1 5 10 15
   Cys